# EUROPEAN PATENT APPLICATION

(11) **EP 4 751 709 A1**
(43) Date of publication of application: **03.06.2026**
(21) Application number: 24845516.4
(22) Date of filing: 18.07.2024
(51) Int. Cl.: A61K 8/37, A61Q 19/00

(54) **ESTER, BASE AGENT FOR COSMETIC, AND COSMETIC**

(30) Priority: 25.07.2023 JP 2023121077
(71) Applicant: NOF Corporation, Shibuya-ku Tokyo 150-6019 (JP); Shiseido Company, Ltd., Chuo-ku Tokyo 104-0061 (JP)
(72) Inventor: URABE, Atsumi, Amagasaki-shi, Hyogo 660-0095 (JP); SHIMIZU, Yutaro, Amagasaki-shi, Hyogo 660-0095 (JP); ODA, Kazuhiro, Amagasaki-shi, Hyogo 660-0095 (JP); ITO, Yuji, Tokyo 104-0061 (JP); ISHIKAWA, Riko, Tokyo 104-0061 (JP); IETANI, Saori, Tokyo 104-0061 (JP)
(74) Representative: J A Kemp LLP
(86) International application number: PCT/JP2024/025761
(87) International publication number: WO 2025/023137

(57) **Abstract**

The present invention provides an ester synthesized from component A which is at least one kind selected from divalent alcohols having 2 to 8 carbon atoms, component B which is at least one kind selected from divalent carboxylic acids having 2 to 10 carbon atoms, and component C which is at least one kind selected from hydroxycarboxylic acids having 4 to 6 carbon atoms and two or more carboxy groups.

## Description

### [Technical Field]

The present invention relates to an ester, a base for cosmetics composed of the aforementioned ester, and a cosmetic containing the aforementioned base for cosmetics.

### [Background Art]

In order to maintain healthy skin, moisture retention is essential and many cosmetics aiming at moisturization have been developed. In cosmetics, hydrocarbon oils such as petrolatum, liquid paraffin, and the like, and ester oils such as neopentyl glycol dicaprate, glyceryl tri(2-ethylhexanoate), and the like are used to moisturize the skin and enhance flexibility of the skin. However, hydrocarbon oils such as some waxes and the like have problems in the feeling of use, such as stickiness, oiliness, and the like. Therefore, ester oils with a less oily feeling are preferably used. As an ester oil with good moisturizing property and good feeling of use, for example, Patent Literature 1 discloses an ester composed of a polyhydric alcohol, a fatty acid having 8 to 30 carbon atoms, and a dibasic carboxylic acid having 12 to 30 carbon atoms, such as an ester synthesized from glycerin, behenic acid, and eicosanedioic acid (hereinafter referred to as "glyceryl behenate eicosadioate") and the like.

Recently, there is a growing preference for more environmentally friendly products, and bases made from natural materials are preferred as the bases used in those products. In particular, there is a demand for a base that is made from natural materials, offers more superior moisturizing effects, and is free of a sticky feeling. Particularly, a moisturizing component that is soluble in oil and water but shows an emollient effect like oil, and can be incorporated into formulations for a wide range of applications has been desired. As a moisturizing base using natural materials, for example, malic acid which is one kind of hydroxycarboxylic acid is widely used in cosmetics because it is superior in safety and stability. For example, Patent Literature 2 discloses diisostearyl malate as a derivative of malic acid.

However, even though esters such as glyceryl behenate eicosandioate and diisostearyl malate have function as oil agents and moisturizing property, the application range thereof is limited because they are insoluble in water.

### [Citation List]

### [Patent Literature]

[Patent Literature 1]
   JP 2009-19049 A
[Patent Literature 2]
   JP 2020-2055 A

### [Summary of Invention]

### [Technical Problem]

The present invention has been made in consideration of the above-mentioned situation, and aims to provide an ester that has good solubility in water and oil and is free of a sticky feeling.

### [Solution to Problem]

The present invention capable of achieving the above-mentioned purpose is as follows.
[1] An ester synthesized from component A which is at least one kind selected from divalent alcohols having 2 to 8 carbon atoms;
   component B which is at least one kind selected from divalent carboxylic acids having 2 to 10 carbon atoms; and
   component C which is at least one kind selected from hydroxycarboxylic acids having 4 to 6 carbon atoms and two or more carboxy groups.
[2] A base for cosmetics, consisting of the ester of the aforementioned [1].
[3] A cosmetic comprising the base for cosmetics of the aforementioned [2].

### [Advantageous Effects of Invention]

According to the present invention, an ester that has good solubility in water and oil and is free of a sticky feeling is obtained.

### [Description of Embodiments]

Preferred embodiments of the present invention are described in detail below.

Respective descriptions in the present specification can be combined with each other unless it is clear that they cannot be combined. In the present specification, numerical ranges defined using "to" include numerical values at both ends (upper limit and lower limit). For example, "2 to 8" means "2 or more and 8 or less."

### <Ester>

The ester of the present invention is synthesized from component A which is at least one kind selected from divalent alcohols having 2 to 8 carbon atoms;
component B which is at least one kind selected from divalent carboxylic acids having 2 to 10 carbon atoms; and
component C which is at least one kind selected from hydroxycarboxylic acids having 4 to 6 carbon atoms and two or more carboxy groups.

Examples of component A include ethylene glycol, 1,2-propanediol, 1,3-propanediol, 1,2-butanediol, 1,3-butanediol, 1,4-butanediol, 2,3-butanediol, 1,2-pentanediol, 1,5-pentanediol, 1,2-hexanediol, 1,6-hexanediol, neopentylglycol, 1,2-octanediol, 1,6-octanediol, and the like.

In view of the solubility of the ester of the present invention in water and feeling of use, component A is preferably at least one kind selected from divalent alcohols having 2 to 6 carbon atoms,
more preferably at least one kind selected from divalent alcohols having 2 to 4 carbon atoms,
further preferably at least kind selected from linear divalent alcohols having 3 to 4 carbon atoms,
particularly preferably 1,3-propanediol and/or 1,4-butanediol, most preferably 1,3-propanediol.

Examples of component B include oxalic acid, malonic acid, succinic acid, maleic acid, glutaric acid, adipic acid, sebacic acid, azelaic acid, and the like.

In view of the solubility of the ester of the present invention in water and feeling of use, component B is preferably at least one kind selected from divalent carboxylic acids having 2 to 8 carbon atoms,
more preferably at least one kind selected from divalent carboxylic acids having 2 to 6 carbon atoms,
further preferably at least one kind selected from divalent saturated carboxylic acids having 4 to 6 carbon atoms,
particularly preferably succinic acid and/or adipic acid.

Examples of component C include malic acid, tartaric acid, citric acid, and the like. The number of carboxy groups present in one molecule of component C is preferably 2 or 3.

In view of the feeling of use, component C is preferably at least one kind selected from hydroxycarboxylic acids having 4 to 6 carbon atoms, one hydroxy group, and two or more carboxy groups,
more preferably at least one kind selected from hydroxycarboxylic acids having 4 to 6 carbon atoms, one hydroxy group, and two or three carboxy groups,
further preferably malic acid and/or citric acid,
particularly preferably malic acid.

From the viewpoint of suppression of a sticky feeling and water solubility of the ester of the present invention, the proportion of constituent components derived from component A (hereinafter referred to as "A_{mol%}) is preferably 50 to 65 mol%, the proportion of constituent components derived from component B (hereinafter referred to as "B_{mol%}) is preferably 1 to 25 mol%, and the proportion of constituent components derived from component C (hereinafter referred to as "C_{mol%})" is preferably 25 to 34 mol%. The basis for A_{mol%}, B_{mol%}, and C_{mol%} is the total of the constituent components derived from components A to C.

From the viewpoint of suppression of a sticky feeling and water solubility of the ester of the present invention, the proportion of A_{mol%} is more preferably 55 to 65 mol%. From the same viewpoint, B_{mol%} is more preferably 10 to 25 mol%, further preferably 10 to 20 mol%. From the same viewpoint, C_{mol%} is more preferably 25 to 32 mol%, further preferably 25 to 29 mol%. From the same viewpoint, it is more preferable that A_{mol%} is 50 to 65 mol%, B_{mol%} is 10 to 25 mol%, and C_{mol%} is 25 to 32 mol%, and it is further preferable that A_{mol%} is 55 to 65 mol%, B_{mol%} is 10 to 20 mol%, and C_{mol%} is 25 to 29 mol%.

A_{mol%}, B_{mol%}, and C_{mol%} are values calculated by analyzing the ¹H NMR (frequency: 400 MHz, solvent: deuterated methanol) chart of the ester of the present invention.

From the viewpoint of suppressing a sticky feeling and water solubility, the hydroxyl value of the ester of the present invention is preferably 100 to 500 mg KOH/g, more preferably 200 to 450 mg KOH/g, further preferably 300 to 400 mg KOH/g.

From the viewpoint of suppressing a sticky feeling, the kinematic viscosity of the ester of the present invention at 40°C is preferably 100 to 600 mm²/s, more preferably 150 to 450 mm²/s, further preferably 150 to 300 mm²/s.

The ester of the present invention can be produced by the esterification reaction of component A, component B, and component C. The esterification reaction is well known to those skilled in the art, and those skilled in the art can produce the ester of the present invention by appropriately setting the esterification reaction conditions.

The esterification reaction is generally performed while removing water from the system to promote the reaction. The esterification reaction may be performed under normal pressure or under reduced pressure. The temperature of esterification reaction is, for example, 100 to 200°C. To obtain an ester with a superior color, the temperature of of the esterification reaction is preferably 100 to 160°C, more preferably 120 to 150°C.

The esterification reaction may be performed in the presence of a catalyst (e.g., Bronsted acid catalyst, Lewis acid catalyst) or without a catalyst.

The progress of the esterification reaction can be monitored by measuring the acid value of the reaction mixture. The obtained ester is preferably purified by a known method. For example, free carboxylic acid remaining in the reaction mixture may be removed using an alkaline aqueous solution, or the reaction mixture may be subjected to distillation, steam deodorization, activated carbon treatment, and the like in order to reduce odor and improve stability.

### <Base for cosmetics and cosmetics>

The present invention provides a base for cosmetics which consists of the ester of the present invention. As used herein, the "base for cosmetics" refers to the base component that constitutes a cosmetic. The present invention also provides a cosmetic containing the base for cosmetics of the present invention.

The content of the base for cosmetics of the present invention in the cosmetic of the present invention is preferably 0.001 to 90% by mass, more preferably 0.1 to 50% by mass, further preferably 0.1 to 20% by mass, particularly preferably 0.1 to 10% by mass, with respect to the whole cosmetic.

The cosmetic of the present invention may contain other components different from the aforementioned base for cosmetics of the present invention. Examples of other component include, but are not limited to, powder component, liquid fat and oil, solid fat and oil, wax, hydrocarbon, higher fatty acid, higher alcohol, ester different from the base for cosmetics of the present invention, silicone, anionic surfactant, cation surfactant, amphoteric surfactant, nonionic surfactant, water-soluble polymer, thickener, film forming agent, ultraviolet absorber, metal ion sequestrant, lower alcohol, polyhydric alcohol, saccharide, amino acid, organic amine, polymer emulsion, pH adjuster, skin nutrient, vitamin, antioxidant, antioxidant aid, flavor, water, and the like. Only one kind of other component may be used, or two or more kinds thereof may be used in combination.

The form of the cosmetic of the present invention is not particularly limited, and may be any of, for example, solution, emulsion, powder, water-oil two layers, water-oil-powder three layers, gel, paste, mist, spray, mousse, roll-on, and stick form. The cosmetic of the present invention may also be used in the form of being impregnated into or applied to a sheet of a nonwoven fabric or the like.

The product form of the cosmetic of the present invention is not particularly limited and, for example, facial cosmetics such as skin lotion, milky lotion, cream, gel, pack, water balm, and the like; makeup cosmetics such as makeup base, foundation, lip stick, eye shadow, and the like; sunscreen cosmetic (sunscreen agent); body cosmetics such as deodorant, deodorant spray, deodorant sheet, and the like; fragranced cosmetic; skin or facial cleansers such as makeup remover, body shampoo, and cleansing lotion, and the like; hair cosmetics such as hair liquid, hair tonic, hair wax, hair conditioner, hair treatment, shampoo, rinse, hair growth agent, and the like; ointment, and the like can be mentioned.

### [Example]

The present invention is described in more detail below using examples, but the present invention is not limited to them.

### <Synthesis of ester>

### (Example 1)

1,3-Propanediol (200 g, 2.63 mol), adipic acid (34.9 g, 0.24 mol), and malic acid (134.1 g, 0.56 mol) were charged into a four-necked flask. Under a nitrogen atmosphere, the reaction water was distilled off at 130°C and normal pressure, the acid value was measured every hour, and the reaction was continued until the rate of decrease in the acid value per hr reached 0.5 or less. Then, the acid value was measured every hour at the same temperature under a reduced pressure of 1 to 5 kPa, and the reaction was continued until the rate of decrease in the acid value per hr reached 0.5 or less to obtain a crude ester. Thereafter, ethyl acetate (350 g) was charged, the mixture was washed with water to remove excess 1,3-propanediol, and ethyl acetate was then removed by distillation under reduced pressure. Then, acid clay and silica-alumina-based adsorbent were each added to amounts of 1.0% by mass of the ester, and adsorption treatment was performed. The temperature, pressure, and time of the adsorption treatment were 100°C, 1 to 5 kPa, and 2 hr. Finally, filtration was performed using a 1 micron filter to obtain ester 1.

### (Example 2)

1,3-Propanediol (230 g, 3.02 mol), succinic acid (59.5 g, 0.50 mol), and citric acid (64.5 g, 0.34 mol) were charged in a four-necked flask, and the subsequent steps were performed in the same manner as in Example 1 to obtain ester 2.

### (Comparative Example 1)

1,3-Propanediol (220 g, 2.89 mol) and malic acid (129.2 g, 0.96 mol) were charged in a four-necked flask, and the subsequent steps were performed in the same manner as in Example 1 to obtain ester 3.

### (Comparative Example 2)

1,3-Propanediol (200 g, 2.63 mol) and adipic acid (129.1 g, 0.88 mol) were charged in a four-necked flask, and the subsequent steps were performed in the same manner as in Example 1 to obtain ester 4.

### <Analysis of ester>

The obtained esters were analyzed according to the following method. The results are shown in Table 1.

### (Composition)

¹H NMR measurement of the obtained ester was performed, and A_{mol%}, B_{mol%}, and C_{mol%} were calculated.

### (Hydroxyl value)

The hydroxyl value of the obtained ester was measured in accordance with JIS K0070.

### (Kinematic viscosity)

The kinematic viscosity of the obtained ester was measured in accordance with JIS K2283.

### (Solubility in water)

A 50% by mass aqueous solution of the obtained ester was prepared, and the appearance thereof was visually confirmed. A uniform appearance was evaluated as ○, and a heterogeneous appearance was evaluated as ×.

### (Solubility in olive oil)

A 1% by mass olive oil solution of the obtained ester was prepared, and the appearance thereof was visually confirmed. A uniform appearance was evaluated as ○, and a heterogeneous appearance was evaluated as ×.

### (Sticky feeling)

The obtained ester (about 1 g) was blended into the skin of twenty women (22 to 40 years of age) as panelists, and sticky feeling was evaluated using the following rating and criteria. Esters evaluated as "○" or "⊙" were determined to be the base for cosmetics with no sticky feeling.

### (Rating)

2 points: no sticky feeling after blended into skin
1 point: slight sticky feeling after blended into skin
0 point: sticky feeling after blended into skin

### (Criteria)

⊙: total score is 30 or more
○: total score is 20 to 29 points
×: total score is 19 or less

**[Table 1]**

| | | Example 1 | Example 2 | Comparative Example 1 | Comparative Example 2 |
|---|---|---|---|---|---|
| | | ester 1 | ester 2 | ester 3 | ester 4 |
| A_{mol%} (mol%) | | 61 | 55 | 57 | 58 |
| B_{mol%} (mol%) | | 12 | 20 | 0 | 42 |
| C_{mol%} (mol%) | | 27 | 25 | 43 | 0 |
| hydroxyl value (mg KOH/g) | | 359 | 323 | 203 | 172 |
| kinematic viscosity (40°C, mm²/s) | | 202 | 428 | 708 | 122 |
| solubility | water | ○ | ○ | ○ | × |
| | olive oil | ○ | ○ | ○ | ○ |
| sticky feeling | | ⊙ (33) | ○ (25) | × (8) | ⊙ (36) |

From the results in Table 1, the ester of the present invention has good solubility in water and oil, is free of a sticky feeling, and is superior in feeling of use.

### <Formulation Examples of cosmetics>

Formulation Examples of cosmetics using the ester of the present invention (i.e., ester 1 or 2) as a base for cosmetics are described in the following. The numerical value for each component below (e.g., "10" in "Glycerin:10") indicates the amount of the component (% by mass).

### [Formulation Example 1] oil-in-water milky lotion

Water: balance
Glycerin: 10
Butylene Glycol: 5
Dipropylene Glycol: 5
Dimethicone: 3
Diglycerine: 1
PEG-240/HDI Copolymer Bis-Decyltetradeceth-20 Ether: 1
Sodium Chloride: 0.5
Gellan Gum: 0.2
Polyquaternium-51: 0.01
Aspartic Acid: 0.1
Camellia Sinensis Leaf Extract: 0.01
Ester 1 or 2: 0.5
Sodium Acetylated Hyaluronate: 0.01
Trimethylsiloxysilicate: 0.1
Soluble Collagen: 0.01
Sodium Citrate: 0.1
Sodium Metaphosphate: 0.1
Acrylates/C10-30 Alkyl Acrylate Crosspolymer: 0.05
Ethanol: 0.5
Potassium Hydroxide: 0.03
Citric Acid: 0.1
Sodium Metabisulfite: 0.1
BHT: 0.01
Phenoxyethanol: 0.6
Chlorphenesin: 0.2
Flavor: 0.05

### [Formulation Example 2] water-in-oil cream

Water: balance
Glycerin: 10
Butylene Glycol: 6
Retinol: 1
Isododecane: 5
Isohexadecane: 5
Dimethicone: 5
Cetyl Ethylhexanoate: 5
Hydrogenated Polydecene: 4
Disteardimonium Hectorite: 2
PEG-9 Polydimethylsiloxyethyl Dimethicone: 1.5
PEG-150: 0.5
Ester 1 or 2: 0.5
Phytosteryl Macadamiate: 1
Stearyl Glycyrrhetinate: 0.1
Tocopheryl Acetate: 0.1
Hydroxyproline: 0.05
Sodium Acetylated Hyaluronate: 0.01
Saccharomyces Ferment Lysate Filtrate: 0.1
Rubus Idaeus (Raspberry) Fruit Extract: 0.01
PEG-8 Diisostearate: 0.5
Sodium Citrate: 0.1
Trisodium EDTA: 0.1
Citric Acid: 0.05
Sodium Metaphosphate: 0.02
Syzygium Jambos Leaf Extract: 0.01
Tocopherol: 0.01
Phenoxyethanol: 0.6
MethylParaben: 0.15
EthylParaben: 0.15
Flavor: 0.15
Iron Oxide: 0.001

### [Formulation Example 3] water-in-oil cream

Water: balance
Glycerin: 10
Butylene Glycol: 6
Isododecane: 5
Isohexadecane: 5
Dimethicone: 5
Cetyl Ethylhexanoate: 5
Hydrogenated Polydecene: 4
Disteardimonium Hectorite: 0.5
PEG-9 Polydimethylsiloxyethyl Dimethicone: 0.5
PEG-150: 0.5
Ester 1 or 2: 0.5
Phytosteryl Macadamiate: 1
Stearyl Glycyrrhetinate: 0.1
Tocopheryl Acetate: 0.1
Hydroxyproline: 0.05
Sodium Acetylated Hyaluronate: 0.01
Saccharomyces Ferment Lysate Filtrate: 0.1
Rubus Idaeus (Raspberry) Fruit Extract: 0.01
PEG-8 Diisostearate: 0.2
Sodium Citrate: 0.1
Trisodium EDTA: 0.1
Citric Acid: 0.05
Sodium Metaphosphate: 0.02
Syzygium Jambos Leaf Extract: 0.01
Tocopherol: 0.01
Phenoxyethanol: 0.6
MethylParaben: 0.15
EthylParaben: 0.15
Flavor: 0.15
Iron Oxide: 0.001

### [Formulation Example 4] oil-in-water gel containing oil-based capsules

Water: balance
Glycerin: 10
Dipropylene Glycol: 10
Ethanol: 3
Diglycerine: 1
PEG-20: 1.5
Pentaerythrityl Tetraethylhexanoate: 2
Dimethicone: 2
Sodium Acrylate/Sodium Acryloyldimethyl Taurate Copolymer: 1
Ester 1 or 2: 1
Candelilla Wax: 0.6
Saccharomyces Ferment Lysate Filtrate: 0.01
Aspartic Acid: 0.01
Squalane: 0.5
Lysine HCl: 0.01
Phytosteryl Macadamiate: 0.1
Sesamum Indicum (Sesame) Seed Oil: 0.1
Simmondsia Chinensis (Jojoba) Seed Oil: 0.1
Sodium Acetylated Hyaluronate: 0.01
Betaine: 0.01
Glycine Soja (Soybean) Seed Extract: 0.01
Soluble Collagen: 0.01
Isohexadecane: 1
PEG-400: 0.5
Polysorbate 80: 0.2
Sorbitan Oleate: 0.05
Talc: 0.5
Disodium EDTA: 0.1
Acrylates/C10-30 Alkyl Acrylate Crosspolymer: 0.01
Dextrin Palmitate: 0.002
Calcium Stearate: 0.01
Potassium Hydroxide: 0.001
PEG-9 Polydimethylsiloxyethyl Dimethicone: 0.01
Tetrahydrotetramethylcyclotetrasiloxane: 0.01
Citric Acid: 0.001
Sodium Phosphate: 0.001
Tetradecene: 0.001
Butylene Glycol: 1
Tocopherol: 0.01
Phenoxyethanol: 0.35
Flavor: 0.05
Iron Oxide: 0.01

### [Formulation Example 5] skin lotion

Water: Balance
Ethanol: 5
Glycerin: 5
Dipropylene Glycol: 5
Ester 1 or 2: 5
Diglycerine: 1
PEG/PPG-17/4 Dimethyl Ether: 2
Crystalline Cellulose (*1): 2
Betaine: 4
Aspartic Acid: 0.1
Lysine HCl: 0.01
Sodium Acetylated Hyaluronate: 0.1
Glycine Soja (Soybean) Seed Extract: 0.01
Soluble Collagen: 0.1
PPG-13-Decyltetradeceth-24: 1
Carbomer: 0.05
Potassium Hydroxide: 0.05
Disodium EDTA: 0.02
Butylene Glycol: 1
Tocopherol: 0.01
Phenoxyethanol: 0.6
(*1 LeoCrysta C-2SP (manufactured by DKS Co. Ltd.))

### [Formulation Example 6] skin lotion containing liposomes

Water: balance
Dipropylene Glycol: 5
Glycerin: 5
Diglycerine: 3
Propanediol: 4
Ester 1 or 2: 2
Butylene Glycol: 2
Diphenylsiloxy Phenyl Trimethicone: 1
PEG-60 Hydrogenated Castor Oil: 1
Sodium PCA: 0.5
Erythritol: 0.5
Dipotassium Glycyrrhizate: 0.1
Sodium Polyacrylate: 0.02
Hydrogenated Lecithin: 0.01
Magnesium Chloride: 0.01
Angelica Acutiloba Root Extract: 0.01
Olea Europaea (Olive) Leaf Extract: 0.01
Calcium Chloride: 0.01
Serine: 0.01
Sodium Acetylated Hyaluronate: 0.01
Oryzanol: 0.001
Hamamelis Virginiana (Witch Hazel) Leaf Extract: 0.01
Chaenomeles Sinensis Fruit Extract: 0.01
Zingiber Aromaticus Extract: 0.01
Rosa Roxburghii Fruit Extract: 0.01
Polyquaternium-51: 0.01
Bupleurum Falcatum Root Extract: 0.01
Isostearyl Alcohol: 1
Polyglyceryl-2 Diisostearate: 0.5
PEG-20: 1
Sodium Citrate: 0.1
Ethanol: 1
Disodium EDTA: 0.05
Citric Acid: 0.05
Phenoxyethanol: 0.3
Flavor: 0.1

### [Formulation Example 7] water-in-oil cream

Water: balance
Ethanol: 3
Glycerin: 3
Dipropylene Glycol: 5
Cyclohexasiloxane: 5
Cetyl Ethylhexanoate: 5
Hydrogenated Polydecene: 2
Phytosteryl/Octyldodecyl Lauroyl Glutamate: 2
Dimethicone: 2
PPG-3 Dipivalate: 2
Myristyl Myristate: 1
Methyl Methacrylate Crosspolymer: 1
Dimethicone/Vinyl Dimethicone Crosspolymer: 0.6
Erythritol: 0.5
Ester 1 or 2: 0.5
PEG/PPG-17/4 Dimethyl Ether: 0.5
Tocopheryl Acetate: 0.2
Caffeine: 0.1
Sapindus Mukorossi Peel Extract: 0.01
Angelica Keiskei Leaf/Stem Extract: 0.01
Camellia Sinensis Leaf Extract: 0.01
Citrus Junos Seed Extract: 0.01
Ziziphus Jujuba Fruit Extract: 0.01
Eucheuma Serra/Grateloupia Sparsa/Saccharina Angustata/Ulva Linza/Undaria Pinnatifida Extract: 0.01
Curcuma Longa (Turmeric) Rhizome Extract: 0.01
Saccharina Angustata/Undaria Pinnatifida Extract: 0.01
Chlorella Vulgaris Extract: 0.01
Hydrogenated Palm Oil: 1
Behenyl Alcohol: 0.5
Polysorbate 60: 0.5
PEG-30 Phytosterol: 0.5
Ammonium Acryloyldimethyltaurate/VP Copolymer: 0.6
Batyl Alcohol: 0.3
Butylene Glycol: 1
Carbomer: 0.04
Acrylates/C10-30 Alkyl Acrylate Crosspolymer: 0.05
Potassium Hydroxide: 0.03
Disodium EDTA: 0.01
Sodium Metaphosphate: 0.02
Tocopherol: 0.01
Sodium Metabisulfite: 0.1
HDI/Trimethylol Hexyllactone Crosspolymer: 0.01
Silica: 0.1
Phenoxyethanol: 0.3
Flavor: 0.05
Iron Oxide:: 0.01

### [Formulation Example 8] oil-in-water milky lotion

Water: balance
Dipotassium Glycyrrhizate: 0.1
DL-α-Tocopherol Acetate: 0.1
Seaweed Extract: 0.01
Magnesium Chloride: 0.01
Calcium Chloride: 0.01
Lemon Extract: 0.05
Glycerin: 5
Dipropylene Glycol: 5
Ethanol: 4
1,3-Butylene Glycol: 3
Methylpolysiloxane: 3
Behenyl Alcohol: 1.5
α-Olefin Oligomer: 1
Diisostearyl Malate: 2
Pentaerythritol Tetra(2-Ethylhexanoate): 1
Ester 1 or 2: 1
Stearyl Alcohol: 0.5
Sodium N-Methyl-N-Stearoyl Taurate: 0.3
Disodium Edetate: 0.02
Xanthan Gum: 0.1
Sodium Citrate: 0.1
Distearyldimethylammonium Chloride: 0.05
Citric Acid: 0.02
L-Arginine Hydrochloride: 0.01
Isopropanol: 0.01
Angelica Acutiloba Extract: 0.01
dl-α-Tocopherol 2-L-Ascorbic Acid Phosphoric Acid Diester Potassium Salt: 0.01
Sodium Acetylated Hyaluronate: 0.01
Isodonis Extract: 0.01
Perilla Extract: 0.01
Coix Extract: 0.01
Phenoxyethanol: 0.5

### [Formulation Example 9] oil-in-water cream

Water: balance
Cetyl Ethylhexanoate: 7
Glycerin: 5
Ethanol: 1
Nylon-12: 5
Cetearyl Alcohol: 5
Dipropylene Glycol: 5
Caprylic/Capric Triglyceride: 5
Phytosteryl Macadamiate: 3
Glyceryl Stearate: 1.5
Dimethicone: 1
Diisostearyl Malate: 1
Erythritol: 1
Ester 1 or 2: 1
PEG/PPG-17/4 Dimethyl Ether: 1
Tocopheryl Acetate: 0.2
Caffeine: 0.1
Xanthan Gum: 0.02
Sapindus Mukorossi Peel Extract: 0.01
Angelica Keiskei Leaf/Stem Extract: 0.01
Camellia Sinensis Leaf Extract: 0.01
Citrus Junos Seed Extract: 0.01
Ziziphus Jujuba Fruit Extract: 0.01
Eucheuma Serra/Grateloupia Sparsa/Saccharina Angustata/Ulva Linza/Undaria Pinnatifida Extract: 0.01
Curcuma Longa (Turmeric) Rhizome Extract: 0.01
Saccharina Angustata/Undaria Pinnatifida Extract: 0.01
Chlorella Vulgaris Extract: 0.01
Beheneth-20: 0.5
HDI/Trimethylol Hexyllactone Crosspolymer: 0.1
Carbomer: 0.07
Butylene Glycol: 1
Sodium Citrate: 0.07
Trisodium EDTA: 0.05
Potassium Hydroxide: 0.06
Citric Acid: 0.05
Sodium Metabisulfite: 0.01
Sodium Metaphosphate: 0.02
PPG-3 Dipivalate: 0.1
Tocopherol: 0.1
Silica: 0.1
Phenoxyethanol: 0.7
Flavor: 0.1
Iron Oxide:: 0.01

### [Formulation Example 10] oil-in-water milky lotion

Water: balance
Dipotassium Glycyrrhizate: 0.1
Glucosyl Hesperidin: 0.01
Crataegus Monogyna Extract: 0.01
Turmeric Extract: 0.01
Sapindus Mukorossi Extract: 0.001
Inositol: 0.01
Iris Florentina Root Extract: 0.01
Gingko Extract: 0.01
Nasturtium Officinale Extract: 0.01
Water-Soluble Collagen (F): 0.01
Glycerin: 6
1,3-Butylene Glycol: 6
α-Olefin Oligomer: 4
Ethanol: 3
Cetyl 2-Ethylhexanoate: 3
Methylpolysiloxane: 3
Dipropylene Glycol: 2
Polyoxyethylene Glyceryl Isostearate: 1.5
Polyethylene Glycol 1000: 1
Polyoxyethylene Glyceryl Monostearate: 1
Behenyl Alcohol: 0.5
Petrolatum: 0.5
Polyethylene Glycol 20000: 0.5
Stearic Acid: 0.4
Isostearic Acid: 0.3
Behenic Acid: 0.3
Batyl Alcohol: 0.2
Carboxyvinyl Polymer: 0.2
Potassium Hydroxide: 0.2
Ester 1 or 2: 0.5
Succinoglycan: 0.04
Disodium Edetate: 0.03
Phytosteryl/Octyldodecyl Lauroyl Glutamate: 0.1
Sodium Metabisulfite: 0.01
Phenoxyethanol: 0.6
Flavor: 0.05

### [Formulation Example 11] oil-in-water skin lotion

Water: balance
Ethanol: 4
Dipropylene Glycol: 4
Butylene Glycol: 3.5
Glycerin: 3
Diglycerine: 1.5
Ester 1 or 2: 1
Lactobacillus/Rice Ferment: 1
PEG-60 Hydrogenated Castor Oil: 0.3
Diphenylsiloxy Phenyl Trimethicone: 0.3
Erythritol: 0.1
Dipotassium Glycyrrhizate: 0.1
Xanthan Gum: 0.05
Sodium Polyacrylate: 0.005
Citrus Junos Fruit Extract: 0.01
Sodium Acetylated Hyaluronate: 0.01
Hamamelis Virginiana (Witch Hazel) Leaf Extract: 0.01
Carbomer: 0.15
Polyglyceryl-2 Diisostearate: 0.12
Potassium Hydroxide: 0.12
Disodium EDTA: 0.12
Isostearyl Alcohol: 0.1
Isostearic Acid: 0.1
Sodium Citrate: 0.1
Citric Acid: 0.02
Sodium Metabisulfite: 0.01
Phenoxyethanol: 0.6
Flavor: 0.01

### [Formulation Example 12] skin lotion

Water: balance
Nasturtium Officinale Extract: 0.01
Inositol: 0.01
Water-Soluble Collagen (F): 0.01
Lysine Hydrochloride: 0.01
Dipropylene Glycol: 5
Glycerin: 5
Ethanol: 3
Maltitol: 1.5
1,3-Butylene Glycol: 1
Ester 1 or 2: 3
Polyethylene Polypropylene Decyl tetradecyl Ether: 0.2
Carboxyvinyl Polymer: 0.1
Erythritol: 1
Disodium Edetate: 0.02
Magnesium L-Ascorbyl Phosphate: 0.1
Sodium Polyacrylate: 0.01
Sodium Metabisulfite: 0.01
Rosemary Oil: 0.01
Yeast Extract (3): 0.01
L-Arginine Hydrochloride: 0.01
Phenoxyethanol: 0.5
Flavor: 0.02

### [Formulation Example 13] water-in-oil cream

Water: balance
Hydrogenated Polydecene: 12
Glycerin: 6
Mineral Oil: 7
Petrolatum: 6
Butylene Glycol: 5
Paraffin: 4
Polyglyceryl-2 Diisostearate: 2
Squalane: 3
Glyceryl Oleate: 2
Sodium PCA: 1.5
Dipropylene Glycol: 1
Beeswax: 2
Carnosine: 1.2
Polyethylene: 0.5
Tocopheryl Acetate: 0.2
Phytosteryl/Octyldodecyl Lauroyl Glutamate: 0.1
Caffeine: 0.1
Ester 1 or 2: 0.5
Sapindus Mukorossi Peel Extract: 0.1
Uncaria Gambir Extract: 0.1
Angelica Keiskei Leaf/Stem Extract: 0.1
Sanguisorba Officinalis Root Extract: 0.1
Crataegus Monogyna Flower Extract: 0.1
Camellia Sinensis Leaf Extract: 0.1
Sodium Acetylated Hyaluronate: 0.01
Hydroxyproline: 0.1
Citrus Junos Seed Extract: 0.1
Ziziphus Jujuba Fruit Extract: 0.1
Chlorella Vulgaris Extract: 0.1
Eucheuma Serra/Grateloupia Sparsa/Saccharina Angustata/Ulva Linza/Undaria Pinnatifida Extract: 0.1
Sodium Lactate: 0.1
Curcuma Longa. (Turmeric) Rhizome Extract: 0.1
Saccharina Angustata/Undaria Pinnatifida Extract: 0.1
Microcrystalline Wax: 1
Sodium Hydroxide: 0.5
Ethanol: 1
Trisodium EDTA: 0.1
Tocopherol: 0.1
Sodium Metabisulfite: 0.1
PPG-3 Dipivalate: 0.1
Cinnamomum Cassia Bark Extract: 0.1
Carbomer: 0.01
BHT: 0.01
Polysorbate 20: 0.1
Palmitoyl Tripeptide-1: 0.1
Palmitoyl Tetrapeptide-7: 0.1
MethylParaben: 0.01
EthylParaben: 0.01
Flavor: 0.01
Iron Oxide: 0.01

### [Formulation Example 14] paste facial cleanser

Ethanol: 5
Glycerin: 5
1,3-Butylene Glycol: 5
PEG-8: 10
Stearic Acid: 15
Lauric Acid: 5
Myristic Acid: 10
Glyceryl Stearate (SE): 5
Ester 1 or 2: 0.5
Sodium Methyl Cocoyl Taurate: 1
Polyquaternium-7: 15
Potassium Hydroxide: appropriate amount
Disodium EDTA: appropriate amount
Flavor: appropriate amount
Water: balance

### [Formulation Example 15] pump foam facial cleanser

Glycerin: 10
Dipropylene Glycol: 10
Sorbitol: 10
Lauric Acid: 5
Myristic Acid: 1
Arginine Cocoate: 2
Sodium Methyltaurate: 3
Lauryl Betaine: 5
Polyquaternium-7: 0.5
Ester 1 or 2: 0.5
Antioxidant: appropriate amount
Chelating agent: appropriate amount
Flavor: appropriate amount
Water: balance

### [Formulation Example 16] liquid facial cleanser

Glycerin: 25
Dipropylene Glycol: 10
Lauric Acid: 5
Myristic Acid: 1
Palmitic Acid: 1
Arginine Cocoate: 10
Sodium Methyltaurate: 10
Lauryl Betaine: 5
Xanthan Gum: 0.5
Ester 1 or 2: 0.5
Antioxidant: appropriate amount
Chelating agent: appropriate amount
Flavor: appropriate amount
Water: balance

### [Formulation Example 17] paste cleanser

Glycerin: 40
PEG-400: 0.5
Ethylene Glycol Distearate: 1.5
Sodium Lauryl Glycol Acetate: 1
Sodium Cocoyl Glycinate: 20
Arginine Cocoate: 5
Cocamidopropyl Betaine: 5
Ester 1 or 2: 0.5
Potassium Hydroxide: appropriate amount
Polyethylene: 1
Color material: appropriate amount
Disodium EDTA: appropriate amount
Water: balance
Flavor: appropriate amount

### [Formulation Example 18] facial cleanser

PEG-10 Dimethicone: 0.5
Glycerin: 10
PEG-8 Dioleate: 5
PEG-2 Laurate: 1
Sodium Lauryl Glycol Acetate: 5
Sodium Laureth Sulfate: 10
Sodium Methyl Cocoyl Taurate: 1
Cocamidopropyl Betaine: 10
Ester 1 or 2: 0.5
pH Adjuster: appropriate amount
Stabilizer: appropriate amount
Antiseptic: appropriate amount
Flavor: appropriate amount
Water: balance

### [Formulation Example 19] pump foam skin cleanser

Glycerin: 10
1,3-Butylene Glycol: 10
PPG-9 Diglyceryl Ether/PPG-9: 5
Ester 1 or 2: 0.5
Triethylhexanoin: 1
PEG-60 Hydrogenated Castor Oil: 1
PEG-60 Glyceryl Isostearate: 1
Potassium Cocoyl Glutamate: 10
Sodium Cocoamphoacetate: 5
pH Adjuster: appropriate amount
Antiseptic: appropriate amount
Water: balance

### [Formulation Example 20] makeup remover

Ethanol: 5
DecamethylCyclopentasiloxane: 5
1,3-Butylene Glycol: 10
Glyceryl 2-Ethylhexanoate: 5
POE(8) Glyceryl Monoisostearate: 25
POE(20) Glyceryl Monoisostearate: 5
Ester 1 or 2: 0.5
Dipotassium Glycyrrhizate: 0.05
Citric Acid: appropriate amount
Sodium Citrate: appropriate amount
Phenoxyethanol: appropriate amount
Purified water: balance

### [Formulation Example 21] makeup remover

Purified water: 5
Glycerin: 10
Sorbitol: 10
Sucrose Stearate: 2
Sodium Methyl Cocoyl Taurate: 1
PEG-60 Hydrogenated Castor Oil: 1
PEG/PPG-35/40 Dimethyl Ether: 2
Ester 1 or 2: 0.5
Mineral Oil: balance
Cetyl Ethylhexanoate: 10
Dimethicone: 5
Diphenylsiloxy Phenyl Trimethicone: 5
Algin: appropriate amount
Sodium Citrate: appropriate amount
Citric Acid: appropriate amount
Disodium EDTA: appropriate amount

### [Formulation Example 22] makeup remover

Purified water: balance
PEG-400: 5
Butylene Glycol: 10
Mineral Oil: 20
Dimethicone: 5
Triethylhexanoin: 5
Stearyl Alcohol: 0.5
Behenyl Alcohol: 0.5
Ester 1 or 2: 0.5
PEG-60 Glyceryl Isostearate: 1
Potassium Hydroxide: appropriate amount
Phenoxyethanol: appropriate amount
Disodium EDTA: appropriate amount
Acrylates/C10-30 Alkyl Acrylate Crosspolymer: 0.05
Carbomer: 0.5
Flavor: appropriate amount

### [Formulation Example 23] cleansing lotion

Ethanol: 10
Glycerin: 1
Dipropylene Glycol: 5
PEG-8: 5
PEG/PPG-35/40 Dimethyl Ether: 1
Ester 1 or 2: 0.5
PPG-13-decyltetradeceth-24: 1
pH Adjuster: appropriate amount
Flavor: appropriate amount
Chelating agent: appropriate amount
Water: balance

### [Formulation Example 24] 2-layer makeup remover

### (Oil layer)

Isododecane: 10
Dimethicone: 25
Decyltetradecylamine Oxide: 1.5
Ethylhexylglycerin: 1

### (Water layer)

Ethanol: 10
1,3-Butylene Glycol: 10
Ester 1 or 2: 0.5
Lauryl Betaine
Sodium Chloride: appropriate amount
Antiseptic: appropriate amount
pH Adjuster: appropriate amount
Chelating agent: appropriate amount
Water: balance

### [Formulation Example 25] deodorant

PEG-11 Methyl Ether Dimethicone: 0.5
Alcohol: 60
Butylene Glycol: 0.5
Ester 1 or 2: 0.5
PPG-20-Decyltetradeceth-10: 0.5
Aluminum Chlorohydrate: 10
Sodium Metaphosphate: 0.01
Tocopherol: appropriate amount
Flavor: appropriate amount
Menthol: appropriate amount
Benzalkonium Chloride: 0.05
Hydroxypropyl Methylcellulose: 0.1
Polyvinylpyrrolidone: 0.05
Purified water: balance

### [Formulation Example 26] deodorant spray

LPG: 75

### (Stock solution)

Ethanol: balance
Ester 1 or 2: 0.5
Zinc Oxide: 0.5
PEG-10 Dimethicone: 0.05
Diphenylsiloxy Phenyl Trimethicone: 5
Ethylhexyl Palmitate: 0.5
Calcium Stearate: appropriate amount
Silica: 1
Aluminium/Potassium Sulfate: 1
Tricalcium Phosphate, silver mixture: 0.05
Glycerin: 0.5
Menthol: appropriate amount
Menthoxypropanediol: appropriate amount
Cymen-5-ol: 0.01
Flavor: appropriate amount

### [Formulation Example 27] deodorant sheet

The following stock solution is impregnated into non-woven fabric.

### (Stock solution)

Alcohol: 40
Ester 1 or 2: 0.5
PPG-20-Decyltetradeceth-10: 0.05
Talc: 3
Zinc Phenolsulfonate: 2
Lactic Acid: appropriate amount
Sodium Lactate: appropriate amount
Menthol: appropriate amount
Benzalkonium Chloride0.05
Purified water: balance
Flavor: appropriate amount

### [Formulation Example 28] hair treatment

Water: balance
Alcohol: 15
Glycerin: 5
Propylene Glycol: 5
Ester 1 or 2: 0.5
Dimethicone: 12
Aminopropyl Dimethicone: 0.6
Acrylates/C10-30 Alkyl Acrylate Crosspolymer: 0.1
PEG-10 Dimethicone: 0.1
Carbomer: 0.35
Xanthan Gum: 0.1
Hydroxyethyl Urea: 0.2
Aminomethyl Propanol: appropriate amount
Disodium EDTA: appropriate amount
Flavor: appropriate amount

### [Formulation Example 29] hair wax

### Water: balance

Propylene Glycol: 5
Maltitol: 5
Carbomer: 0.1
PEG-240/HDI Copolymer Bis-Decyltetradeceth-20 Ether: 0.5
Ceteth-25: 3
Glyceryl Stearate (SE): 0.5
Hydrogenated Polyisobutene: 5
Microcrystalline Wax: 5
Paraffin: 0.5
Stearic Acid: 3
Cetyl Alcohol: 3
Ester 1 or 2: 0.5
Candelilla Wax: 10
Carnauba Wax: 1
Cetyl Ethylhexanoate: 3
Pentaerythrityl Tetraethylhexanoate: 0.5

### (Methacryloyloxyethyl Carboxybetaine/Alkyl Methacrylate)

Copolymer: 2
VP/VA Copolymer: 0.5
Triethanolamine: appropriate amount
Phenoxyethanol: appropriate amount
Disodium EDTA: appropriate amount
Tocopherol: appropriate amount
Flavor: appropriate amount

### [Formulation Example 30] oil-in-water sunscreen cosmetic

Purified water: balance
Glycerin: 2.5
Dipropylene Glycol: 5
Ester 1 or 2: 0.5
PEG-60 Glyceryl Isostearate: 0.3
PEG-10 Dimethicone: 0.3
Triethanolamine: 0.3
Behenyl Alcohol: 1
Batyl Alcohol: 1.5
Behenic Acid: 0.6
Pentaerythritol Tetra(2-Ethylhexanoate): 2
Polypropylene Glycol (17): 1
Cyclopentasiloxane: 4
Dimethicone (6cst): 1
Ethylhexyl Methoxycinnamate: 7.5
Bis-Ethylhexyloxyphenol Methoxyphenyl Triazine: 2
Xanthan Gum: 0.2
Phenoxyethanol: 0.35
Chlorphenesin: 0.2
Thiotaurine: 0.1
Aminoethanesulfinic Acid: 0.1
Rosa Multiflora Fruit Extract: 0.1
Rosa Centifolia Flower Extract: 0.1
Titanium Oxide: 0.05
Mica: 0.05
Chelating agent: appropriate amount
pH Adjuster: appropriate amount

### [Formulation Example 31] oil-in-water sunscreen cosmetic

### Purified water: balance

Glycerin: 4
Butylene Glycol: 7
PEG/PPG-9/2 Dimethyl Ether: 5
Compound 1 or Compound 2: 0.5
Polyethylene Glycol 300: 1
Succinoglycan: 0.3
Dimethylacrylamide/Sodium Acryloyldimethyltaurate Crosspolymer: 0.3
Sucrose Fatty Acid Ester: 3
PEG-60 Hydrogenated Castor Oil: 2
Diisopropyl Sebacate: 2
Nonvolatile Dimethicone: 2
Cyclomethicone: 12
Triethylhexanoin: 5
Isostearic Acid: 1
Sorbitan Sesquiisostearate: 0.5
Hydrophobic-treated zinc oxide fine particles: 10
Ethylhexyl Methoxycinnamate: 10
Diethylamino Hydroxybenzoyl Hexyl Benzoate: 1
Bis-Ethylhexyloxyphenol Methoxyphenyl Triazine: 1
Spherical urethane resin powder: 3
Chlorphenesin: 0.2
pH Adjuster: appropriate amount
Chelating agent: appropriate amount

### [Formulation Example 32] oil-in-water sunscreen cosmetic

Purified water: balance
Diisopropyl Sebacate: 8
Bis-Ethylhexyloxyphenol Methoxyphenyl Triazine: 1
Isopentyl Trimethoxycinnamate Trisiloxane: 1
Hydrophobic-treated zinc oxide: 5
Succinoglycan: 1
Dimethylacrylamide/Sodium Acryloyldimethyltaurate Crosspolymer: 0.3
Stearoxy Hydroxypropyl Methylcellulose: 0.2
Chlorphenesin: 0.2
Butylene Glycol: 4
Ethanol: 4
Ester 1 or 2: 0.5

### [Formulation Example 33] water-in-oil sunscreen cosmetic

Purified water: balance
Ethanol: 3
Ester 1 or 2: 0.5
Cellulose Gum: 0.1
Glycerin: 2
Isododecane: 5
Isopropyl Myrstate: 5
Diisopropyl Sebacate: 10
PBG/PPG-9/1 Copolymer: 1
Dimethicone (1.5cs): 10
Dimethicone (6cs): 5
Trimethylsiloxysilicate: 1
Dextrin Palmitate: 1
Ethylhexyl Methoxycinnamate: 5
Diethylamino Hydroxybenzoyl Hexyl Benzoate: 1
Bis-Ethylhexyloxyphenol Methoxyphenyl Triazine: 1
Ethylhexyl Salicylate: 3
Hydrophobic-treated titanium oxide fine particles: 5
Hydrophobic-treated zinc oxide fine particles: 10
PEG-9 Polydimethylpolysiloxyethyl Dimethicone: 1
Cetyl PEG/PPG-10/1 Dimethicone: 1
Dimethyldistearyl Ammonium Hectorite: 0.2
Chlorphenesin: 0.1
Isostearic Acid: 0.1
Chelating agent: appropriate amount
Thiotaurine: 0.1
Aminoethanesulfinic Acid: 0.1
Rosa Multiflora Fruit Extract: 0.1
Rosa Centifolia Flower Extract: 0.1
Titanium Oxide: 0.05
Mica: 0.05

### [Formulation Example 34] oil-in-water sunscreen cosmetic

Purified water: balance
Ethanol: 10
Disodium EDTA·H2O: appropriate amount
Carbomer: 0.2
Acrylates/C10-30 Alkyl Acrylate Crosspolymer: 0.1
Xanthan Gum: 0.1
Agar: 0.1
Glycerin: 3
Butylene Glycol: 5
Ester 1 or 2: 1
Core-corona-type microgel emulsifier Acrylates/Methoxy PEG-90
Methacrylate Crosspolymer (Production Example 9 in
WO2013/094298): 1.5
35% by mass Lauryl Dimethylaminoacetic Acid Betaine aqueous solution: 0.1
Ethylhexyl Methoxycinnamate: 7
Ethylhexyl Triazine: 1
Diethylamino Hydroxybenzoyl Hexyl Benzoate: 1
Bis-Ethylhexyloxyphenol Methoxyphenyl Triazine: 1
Distearyldimethylammonium chloride-treated silica-treated titanium dioxide: 4
Dimethicone: 5
Caprylyl Methicone: 2
Diisopropyl Sebacate: 5
C12-15 Alkyl Benzoate: 3
Dextrin Palmitate/Ethylhexanoate: 1
Alumina-treated titanium oxide: 1
Flavor: appropriate amount
Potassium Hydroxide: 0.15
Phenoxyethanol: 0.5
Silica: 1

### [Formulation Example 35] oil-in-water sunscreen cosmetic

Purified water: balance
Ethanol: 8
Disodium EDTA: appropriate amount
Sodium Hexametaphosphate: appropriate amount
Citric Acid: appropriate amount
Sodium Citrate: appropriate amount
Japanese Cherry Leaf Extract: 1
V-E Acetate: 1
Hamamelis Virginiana: 1
Green Tea Extract: 1
Dimethylacrylamide/Sodium Acryloyldimethyltaurate Copolymer: 0.3
Succinoglycan: 0.1
Stearoxy Hydroxypropyl Methylcellulose: 0.1
Glycerin: 3
Butylene Glycol: 5
Ester 1 or 2: 1
Polyoxyethylene Hydrogenated Castor Oil (60 mol): 1.5
Ethylhexyl Methoxycinnamate: 5
Ethylhexyl Triazine: 2
Diethylamino Hydroxybenzoyl Hexyl Benzoate: 2
Bis-Ethylhexyloxyphenol Methoxyphenyl Triazine: 1
Dimethicone: 6
Isododecane: 3
Dextrin Palmitate/Ethylhexanoate: 1
Hydrophobic-treated silica-coated zinc oxide fine particles: 15
Alumina-treated titanium oxide: 1
Isostearic Acid: 0.3
Sorbitan Sesquiisostearate: 0.3
Flavor: appropriate amount
Silica: 1

### [Formulation Example 36] water-in-oil sunscreen cosmetic

Purified water: balance
Ethanol: 8
EDTA·3Na: appropriate amount
Common salt: appropriate amount
Sodium Metabisulfite: appropriate amount
Glycerin: 2
Ester 1 or 2: 0.5
Isododecane: 3
Glyceryl Tri(2-Ethylhexanoate): 5
Isopropyl Myrstate: 5
Diisopropyl Sebacate: 5
Dimethicone: 6
Trisiloxysilicate: 1
Dextrin Palmitate: 0.5
Homosalate: 5
Diethylamino Hydroxybenzoyl Hexyl Benzoate: 0.5
Bis-Ethylhexyloxyphenol Methoxyphenyl Triazine: 1
Butyl Methoxydibenzoylmethane: 2
Ethylhexyl Salicylate: 5
Hydrophobic-treated titanium oxide fine particles: 3
Hydrophobic-treated zinc oxide fine particles: 7
Methyl Methacrylate Crosspolymer: 3
Silica: 2
Hydrophobic-treated silica: 5
PEG-9 Polydimethylpolysiloxyethyl Dimethicone: 1
Lauryl PEG-9 Polydimethylpolysiloxyethyl Dimethicone: 2
Dimethyldistearyl Ammonium Hectorite: 0.5
Isostearic Acid: 0.5
Sorbitan Sesquiisostearate: 0.5
Tocopherol: appropriate amount
Flavor: appropriate amount

### [Formulation Example 37] lip stick

Polyethylene: 7
Microcrystalline Wax: 1
Candelilla Wax: 1
Petrolatum: 5
Macadamia Nut Oil: 21.92
Glyceryl Triisostearate: 12
Diisostearyl Malate: 10
Phytosteryl/Octyldodecyl Lauroyl Glutamate: 10
Hydrogenated Polyisobutene: 18.5
Diphenylsiloxy Phenyl Trimethicone: 5
Butyrospermum Parkii (Shea) Butter: 0.1
Ethylhexyl Methoxycinnamate: 7.5
Pentaerythrityl Tetraethylhexanoate: 0.2
Ester 1 or 2: 0.5
Color material: 0.05
Dibromofluorescein (SUNCHEMICAL): 0.1
Tetrabromofluorescein (Kohnstamm): 0.1
Tetrachlorotetrabromofluorescein: 0.1
Tocopheryl Acetate: 0.05
Dimethicone/Vinyl Dimethicone Crosspolymer: 0.01
Royal Jelly Extract: 0.05
Titanated Mica: 0.03
Silicic Anhydride: 0.01
Titanium Oxide: 0.01
Tocopherol: 0.05
Simethicone: 0.02

### [Formulation Example 38] lip stick

Polyethylene: 7
Microcrystalline Wax: 1
Candelilla Wax: 1
Petrolatum: 5
Macadamia Nut Oil: 21.72
Glyceryl Triisostearate: 11
Diisostearyl Malate: 9
Phytosteryl/Octyldodecyl Lauroyl Glutamate: 9
Hydrogenated Polyisobutene: 17.3
Diphenylsiloxy Phenyl Trimethicone: 4.5
Butyrospermum Parkii (Shea) Butter: 0.1
Ethylhexyl Methoxycinnamate: 7.5
Pentaerythrityl Tetraethylhexanoate: 0.2
Ester 1 or 2: 0.5
Color material: 0.05
Dibromofluorescein (SUNCHEMICAL): 0.1
Tetrabromofluorescein (Kohnstamm): 0.1
Tetrachlorotetrabromofluorescein: 0.1
Tocopheryl Acetate: 0.05
Dimethicone/Vinyl Dimethicone Crosspolymer: 0.01
Royal Jelly Extract: 0.05
Titanated Mica: 0.03
Silicic Anhydride: 0.01
Titanium Oxide: 0.01
Tocopherol: 0.05
Simethicone: 0.02

### [Formulation Example 39] lip stick

Polyethylene: 7
Microcrystalline Wax: 1
Candelilla Wax: 1
Petrolatum: 5
Macadamia Nut Oil: 17.39
Glyceryl Triisostearate: 9.3
Diisostearyl Malate: 7.8
Phytosteryl/Octyldodecyl Lauroyl Glutamate: 7.8
Hydrogenated Polyisobutene: 14.4
Diphenylsiloxy Phenyl Trimethicone: 4
Butyrospermum Parkii (Shea) Butter: 0.1
Ethylhexyl Methoxycinnamate: 7.5
Pentaerythrityl Tetraethylhexanoate: 5
Ester 1 or 2: 0.5
Color material: 12
Dibromofluorescein (SUNCHEMICAL): 0.1
Tetrabromofluorescein (Kohnstamm): 0.1
Tetrachlorotetrabromofluorescein: 0.1
Tocopheryl Acetate: 0.05
Dimethicone/Vinyl Dimethicone Crosspolymer: 0.01
Royal Jelly Extract: 0.05
Titanated Mica: 0.01
Silicic Anhydride: 0.01
Titanium Oxide: 0.01
Tocopherol: 0.05
Simethicone: 0.02

### [Formulation Example 40] lip stick

Polyethylene: 7
Microcrystalline Wax: 1
Candelilla Wax: 1
Petrolatum: 5
Macadamia Nut Oil: 16.29
Glyceryl Triisostearate: 8.5
Diisostearyl Malate: 7.1
Phytosteryl/Octyldodecyl Lauroyl Glutamate: 7.1
Hydrogenated Polyisobutene: 13.2
Diphenylsiloxy Phenyl Trimethicone: 3.6
Butyrospermum Parkii (Shea) Butter: 0.1
Ethylhexyl Methoxycinnamate: 7.5
Pentaerythrityl Tetraethylhexanoate: 5
Ester 1 or 2: 0.5
Color material: 12
Dibromofluorescein (SUNCHEMICAL): 0.1
Tetrabromofluorescein (Kohnstamm): 0.1
Tetrachlorotetrabromofluorescein: 0.1
Tocopheryl Acetate: 0.05
Dimethicone/Vinyl Dimethicone Crosspolymer: 0.01
Royal Jelly Extract: 0.05
Titanated Mica: 0.01
Silicic Anhydride: 0.01
Titanium Oxide: 0.01
Tocopherol: 0.05
Simethicone: 0.02

### [Formulation Example 41] makeup base

Water: balance
Ethanol: 5
Glycerin: 3
Butylene Glycol: 5
Ester 1 or 2: 0.5
Succinoglycan: 0.3
Cellulose Gum: 0.3
Dimethylacrylamide/Sodium Acryloyldimethyltaurate Crosspolymer: 0.3
Polyglyceryl-10 Stearate: 3
Sorbitan Sesquiisostearate: 1
Polyglyceryl-6 Polyricinoleate: 0.5
C9-12 Alkane/Coco-Caprylate/Caprate: 10
Isopropyl Myristate: 8
Caprylic/Capric Triglyceride: 8
Zinc Oxide/Dextrin Palmitate: 15
Titanium Oxide/Aluminum Hydroxide/Stearic Acid: 5
Silica: 1
Phenoxyethanol: appropriate amount
Citric Acid: appropriate amount
Sodium Citrate: appropriate amount
Disodium EDTA: appropriate amount

### [Formulation Example 42] makeup base

PEG-10 Dimethicone: 3.5
Lauryl PEG-9 Polydimethylsiloxyethyl Dimethicone: 2
PEG-10 Hydrogenated Castor Oil: 2
Ethylhexyl Salicylate: 10
Isohexadecane: 10
Hydrogenated Didecene: 20
Disteardimonium Hectorite: 2
Fatty acid-treated titanium oxide: 4.5
Silica: 5
Silicone-treated titanium oxide: 4.5
Silicone-treated red iron oxide: 0.35
Silicone-treated yellow iron oxide: 0.75
Silicone-treated black iron oxide: 0.015
Water: balance
Trisodium EDTA: 0.2
Glycerin: 2.5
Dipropylene Glycol: 7
Butylene Glycol: 2
Ester 1 or 2: 0.5
Ethanol: 1
Phenoxyethanol: appropriate amount

### [Formulation Example 43] mask spray

Sugar Cane (Saccharum Officinarum) Extract: 1
Hydroxypropyl Cyclodextrin: 2
Citric Acid (Food): appropriate amount
Sodium Citrate: appropriate amount
Common salt: 0.5
Sodium Metabisulfite: appropriate amount
Ethanol: 54
1,3-Butylene Glycol: 3
Ester 1 or 2: 0.5
Phenoxyethanol: 0.5
Glycerin: 1
Citrus Aurantium Dulcis (Orange) Oil: 0.2

### [Formulation Example 44] cushion foundation

Cyclopentasiloxane: 27
Pentaerythrityl Tetraethylhexanoate: 4
PEG-10 Dimethicone: 3
PEG-9 Polydimethylsiloxyethyl Dimethicone: 1.5
Bis-Butyldimethicone Polyglyceryl-3: 1
Disteardimonium Hectorite: 0.5
Octyl Methoxycinnamate: 6.5
Hydrophobized titanium oxide fine particles: 11
Hydrophobized titanium oxide: 7
Hydrophobized red iron oxide: 0.4
Hydrophobized yellow iron oxide : 0.8
Hydrophobized black iron oxide: 0.01
Spherical powder: 3
Water: balance
Glycerin: 10
Ester 1 or 2: 0.5
Paraben: appropriate amount
Phenoxyethanol: appropriate amount

### [Formulation Example 45] water balm

Agar: 0.1
Tamarindus Indica Seed Gum: 1.2
Paraffin Wax: 1
Dimethylacrylamide/Sodium Acryloyldimethyltaurate Crosspolymer: 0.4
Polyethyleneglycol (40EO) Monostearate: 0.5
Polysorbate 60: 0.5
Sorbitan Tristearate: 0.1
Stearyl Alcohol: 0.1
Behenyl Alcohol: 1.5
Glycerin: 15
Pearly pigment: 10
Citric Acid: appropriate amount
Sodium Citrate: appropriate amount
Antiseptic: appropriate amount
Sodium Metabisulfite: appropriate amount
Propanediol: 3
Butylene Glycol: 3
Ester 1 or 2: 0.5
Perfectly spherical porous silica (average particle size: about 5 µm): 1
Non-porous silica (average particle size: about 7 µm): 1.5
PEG 60 Hydrogenated Castor Oil: 0.1
Ethylhexyl Methoxycinnamate: 4
Sorbitan Sesquiisostearate: 1
Isostearic Acid: 1
Glyceryl Stearate: 1
BHT: 0.05
Isohexadecane: 2.5
Water: balance

### [Formulation Example 46] eye shadow

Talc: balance
Synthetic Phlogopite: 10
Zinc Oxide: 1
Barium Sulfate: 1
Magnesium Myristate: 1
Chlorphenesin: appropriate amount
Vinyl Dimethicone/Methicone Silsesquioxane Crosspolymer: 4
Boron Nitride: 8
Jojoba Esters: 3
CI 73360/Mica: 1
Synthetic Phlogopite/Titanium Oxide/Tin Oxide: 20
Petrolatum/Tocopherol: 2
Diisostearyl Malate: 6
Triethylhexanoin: 2
Ester 1 or 2: 0.5

### [Formulation Example 47] oil-in-water foundation

Water: balance
Ethanol: 5
Glycerin: 2.5
1,3-Butylene Glycol: 7
Ester 1 or 2: 0.5
Stearoxy Hydroxypropyl Methylcellulose: 0.05
Succinoglycan: 0.15
Dimethylacrylamide/Sodium Acryloyldimethyltaurate Crosspolymer: 0.25
PEG-100 Hydrogenated Castor Oil: 0.2
PEG-60 Hydrogenated Castor Oil: 1.5
PPG-17: 1
Dimethicone: 2
Triethylhexanoin: 3
Dimethicone: 17
Isostearic Acid: 0.8
Sorbitan Sesquiisostearate: 0.2
Distearyldimonium Chloride: 0.15
Zinc Oxide: 14
Ethylhexyl Methoxycinnamate: 7
Ethylhixyl Triazone: 0.5
Diethylamino Hydroxybenzoyl Hexyl Benzoate/: 0.5
Bis-Ethylhexyloxyphenol Methoxyphenyl Triazine: 2
Titanium Oxide: 0.5
Sodium Metaphosphate: appropriate amount
Phenoxyethanol: appropriate amount

### [Formulation Example 48] makeup cosmetic (solid water-in-oil emulsion)

DecamethylCyclopentasiloxane: 16
Dimethylpolysiloxane (6cps): 10
Polyoxyalkylene-modified organopolysiloxane: 5
Paraffin Wax (165°F): 10
Bis-PEG-18 Methyl Ether Dimethyl Silane: 0.5
Silicone-treated talc: 16
Silicone-treated spherical silica powder: 5
Calcium stearate-treated zinc oxide: 3
Silicone-treated titanium dioxide: 10
Silicone-treated iron oxide (yellow): 3
Silicone-treated iron oxide (red): 1
Silicone-treated iron oxide (black): 0.1
Ion-exchanged water: balance
Glycerin: 5
Ester 1 or 2: 0.5
Antiseptic: appropriate amount
Flavor: appropriate amount

### [Formulation Example 49] oil-based liquid foundation

Dimethylpolysiloxane: 10
DecamethylCyclopentasiloxane: balance
Isohexadecane: 10
Polyether-modified silicone: 2
Isostearic Acid: 0.5
Polyoxyethylene (10) Isostearyl Ether (HLB 12.4): 0.5
Organically modified bentonite: 0.1
Polymethyl methacrylate spherical powder: 5
Hydrophobic-treated yellow iron oxide: 3
Hydrophobic-treated red iron oxide: 1.5
Hydrophobic-treated black iron oxide: 0.3
Hydrophobic-treated titanium oxide: 12
Ester 1 or 2: 0.5

### [Formulation Example 50] facial cosmetic (aqueous composition)

Water: balance
PEG-240/HDI Copolymer Bis-Decyltetradeceth-20 Ether: 0.9
Ammonium Acryloyldimethyltaurate/VP Copolymer: 0.3
Phenoxyethanol: 0.5
Dipropylene Glycol: 8
Butylene Glycol: 5
Glycerin: 2
Ester 1 or 2: 0.5
Synthetic Phlogopite, Distearyldimonium Chloride: 15
Iron Oxide, Triethoxycaprylylsilane: 0.4
Iron Oxide, Triethoxycaprylylsilane: 0.8
Iron Oxide, Triethoxycaprylylsilane: 0.01

### [Industrial Applicability]

The ester of the present invention has good solubility in water and oil, is free of a sticky feeling, and is useful as a base for cosmetics.

This application is based on a patent application No. 2023-121077 filed in Japan, the contents of which are incorporated in full herein.

## Claims

1. An ester synthesized from
component A which is at least one kind selected from divalent alcohols having 2 to 8 carbon atoms;
component B which is at least one kind selected from divalent carboxylic acids having 2 to 10 carbon atoms; and
component C which is at least one kind selected from hydroxycarboxylic acids having 4 to 6 carbon atoms and two or more carboxy groups.

2. A base for cosmetics, consisting of the ester according to claim 1.

3. A cosmetic comprising the base for cosmetics according to claim 2.
